(19) Europäisches Patentamt
European Patent Office
Office européen des brevets

(11) Veröffentlichungsnummer : **0 355 561 B1**

(12) # EUROPÄISCHE PATENTSCHRIFT

(45) Veröffentlichungstag der Patentschrift : **19.11.92 Patentblatt 92/47**

(21) Anmeldenummer : **89114602.9**

(22) Anmeldetag : **08.08.89**

(51) Int. Cl.[5] : **C09K 19/58,** C07D 307/24, C07D 405/12, C07D 407/12, C07D 409/12, C07D 417/12, C07D 405/14, C07D 407/14, C07D 409/14, C07D 417/14

(54) **Verwendung von optisch aktiven Tetrahydrofuran-2-carbonsäureestern als Dotierstoffe in Flüssigkristallmischungen, diese enthaltende Flüssigkristallmischungen und neue optisch aktive Tetrahydrofuran-2-carbonsäureester.**

(30) Priorität : **13.08.88 DE 3827599**

(43) Veröffentlichungstag der Anmeldung : **28.02.90 Patentblatt 90/09**

(45) Bekanntmachung des Hinweises auf die Patenterteilung : **19.11.92 Patentblatt 92/47**

(84) Benannte Vertragsstaaten : **AT BE CH DE FR GB IT LI NL SE**

(56) Entgegenhaltungen :
**CHEMICAL ABSTRACTS, Band 103, Nr. 3, 22. Juli 1985, Columbus, Ohio, USA THAKAR, K.A. et al. "Synthe- sis and antifungalactivity of 3-furyl- and 3-thienyl- -substituted-1,2-benzi- soxazo- les" Seite 569, Spalte 1, Zu- sammenfassung-Nr. 22 501m**
**CHEMICAL ABSTRACTS, Band 109, Nr. 16, 17. Oktober 1988, Columbus, Ohio, USA SATO- NAKA, HAJIME et al. "Furan derivative liquid- crystal compounds, inter- mediate compounds, and liquid crystal compositions" Seite 727, Spalte 2, Zu- sammenfassung-Nr. 139 802a**
**CHEMICAL ABSTRACTS, Band 94, Nr. 24, 15. Juni 1981, Columbus, Ohio, USA HITACHI, LTD. "Liquid crystal compositions fordisplay devices" Seite 605, Spalte 2, Zu- sammenfas- sung-Nr. 200 881r**
**CHEMICAL ABSTRACTS, Band 103, Nr. 6, 12. August 1985, Columbus, Ohio, USA TAKE- NAKA, SHUNSUKE et al. "The smectic A- smectic Atransition in a homologous series of 4-(4-alkoxyphenoxy- carbonyl)phenyl 5-cy- ano-2- -furancarboxylates" Seite 622, Spalte 1, Zu-sammenfassung-Nr. 46 339r**

(56) Entgegenhaltungen :
**CHEMICAL ABSTRACTS, Band 104, Nr. 12, 24. MUrz 1986, Columbus, Ohio, USA TAKENAKA, S. et al. "The SA-SA transition in a homo- logous series of 4(4-alkoxy- phenoxycarbo- nyl)phenyl 5- -nitro-2-furancarboxylates" Seite 762, Spalte 2, Zu- sammenfassung-Nr. 99976y**

(73) Patentinhaber : **HOECHST AKTIENGESELLSCHAFT**
**Postfach 80 03 20**
**W-6230 Frankfurt am Main 80 (DE)**

(72) Erfinder : **Hemmerling, Wolfgang, Dr.**
**Billtalstrasse 32**
**W-6231 Sulzbach (Taunus) (DE)**
Erfinder : **Dübal, Hans-Rolf, Dr.**
**Heuhohlweg 6**
**W-6240 Königstein/Taunus (DE)**
Erfinder : **Escher, Claus, Dr.**
**Amselweg 3**
**W-6109 Mühtal (DE)**
Erfinder : **Illian, Gerhard, Dr.**
**Rauenthalerweg 32**
**W-6000 Frankfurt am Main 71 (DE)**
Erfinder : **Inoguchi, Yoshio, Dr.**
**4-12-201, Fujicho 4-chome**
**Hohya-shi Tokyo (JP)**
Erfinder : **Müller, Ingrid, Dr.**
**Am Pfingstbrunnen 1**
**W-6238 Hofheim am Taunus (DE)**
Erfinder : **Murakami, Mikio**
**Bahnstrasse 2c**
**W-6240 Königstein/Taunus (DE)**
Erfinder : **Ohlendorf, Dieter, Dr.**
**Am Kühlen Grund 4**
**W-6237 Liederbach (DE)**
Erfinder : **Wingen, Rainer, Dr.**
**Rotkäppchenweg 10**
**W-6234 Hattersheim am Main (DE)**

**EP 0 355 561 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu lang. Dieser Nachteil macht sich insbesondere dann bemerkbar, wenn eine große Anzahl von Bildpunkten angesteuert werden muß, wodurch die Herstellungskosten von Geräten, die größere Flächen enthalten, z.B. Videogeräte, Oszillographen oder Fernseh-, Radar-, EDV- oder Schreibautomaten-Bildschirme, zu hoch werden.

Neben den nematischen und cholesterischen Flüssigkristallen haben seit einigen wenigen Jahren in zunehmendem Maß auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich mit den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLC's grundsätzlich für die obengenannten Anwendungsgebiete, z.B. über eine Matrixansteuerung, gut geeignet.

Einen anderen elektro-optischen Effekt. der als elektrokliner Effekt bezeichnet wird, zeigen orthogonale chirale smektische Phasen, z.B. $S_A^*$, $S_B^*$ , $S_E^*$. Dieser Effekt (S. Garoff et al., Phys. Rev. Lett. 38, 848 (1977)) besteht in einer feldinduzierten Neigung der Moleküle, deren Neigungswinkel $\theta$ sich proportional zum angelegten Feld ändert. Die Moleküle der orthogonalen Phasen können daher der Feldänderung kontinuierlich folgen, und sie können insbesondere einem Wechselfeld bis zu einer Grenzfrequenz $f_G$ folgen, während ferroelektrische Systeme jeweils beim Erreichen einer bestimmten Feldstärke ihren Neigungswinkel sprunghaft ändern und beibehalten, bis ein entsprechendes gegensinniges Feld angelegt wird (bistabiles Schalten).

Beide Effekte, der ferroelektrische, wie der elektrokline, lassen sich je nach ihren speziellen Eigenschaften zum Bau von elektrooptischen Schalt- und Anzeigeelementen ausnutzen. Man benötigt dazu entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische bzw. elektrokline smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A^*$ und $S_C^*$-Phase läßt sich erreichen, wenn die Phasenfolge der Flüssigkristallmischung mit abnehmender Temperatur lautet:

$$\text{Isotrop} \rightarrow N* \rightarrow S_A^* \rightarrow S_C^*.$$

Voraussetzung ist, daß der Pitch (Ganghöhe der Helix) in der N∗-Phase sehr groß (größer 10 µm) oder noch besser völlig kompensiert ist. (T. Matsumoto et al., p. 468-470, Proc. of the 6th Int. Display Research Conf., Japan Display, Sept. 30 - Okt. 2, 1986, Tokyo, Japan; M. Murakami et al., ibid. S.344 - S.347). Dies erreicht man, indem man zu der chiralen Flüssigkristallmischung, die in der N∗-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

Es wurde nun gefunden, daß optisch aktive Tetrahydrofuran-2-carbonsäureester als Dotierstoffe in geneigt smektischen Flüssigkristallphasen schon bei geringen Zumischmengen zu kurzen Schaltzeiten und in orthogonal smektischen Flüssigkristallphasen zu hohen elektroklinen Koeffizienten führen. Die durch die Tetrahydrofuran-2-carbonsäureester in der N∗-Phase induzierte Helix kann in Mischungen vorteilhaft zur gezielten Kompensation der Ganghöhe verwendet werden.

Gegenstand der Erfindung ist daher die Verwendung von optisch aktiven Tetrahydrofuran-2-carbonsäureestern als Dotierstoffe in Flüssigkristallmischungen. Gegenstand der Erfindung sind weiterhin Flüssigkristallsysteme, die optisch aktive Tetrahydrofuran-2-carbonsäureester enthalten sowie neue optisch aktive Tetrahydrofuran-2carbonsäureester. Die gemäß der Erfindung einzusetzenden Tetrahydrofuran-2-carbonsäureester entsprechen der allgemeinen Formel (I)

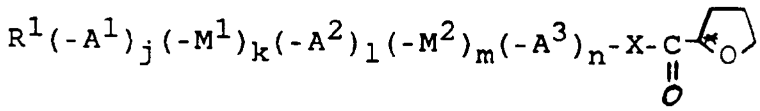 (I)

in der die Symbole und Indices folgende Bedeutung haben: $R^1$

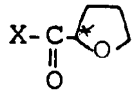

oder ein geradkettiger oder verzweigter

Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, eine oder mehrere nicht benachbarte -$CH_2$-Gruppen durch -O-, -S-, -CO-, -O-CO- und/oder -CO-O- ersetzt sein können, und wobei ein oder mehrere H durch F, Cl, Br oder CN ersetzt sein können

j und l null, 1 oder 2

k und m null oder 1

n null, 1 oder 2

mit folgender Maßgabe: wenn j und/oder l = null sind, ist k = null; wenn n = null ist, ist m = null; die Summe j + l + n ist minimal 1 und maximal 3,

$-A^1$, $-A^2$

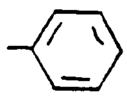 , 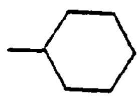 , 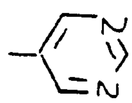 , 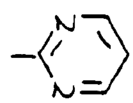 ,

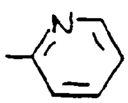 , 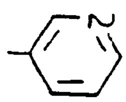 , 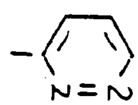 , 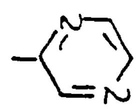 ,

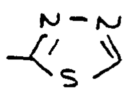 , 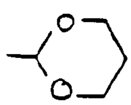 , 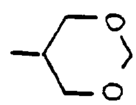 , 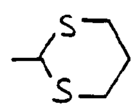 ,

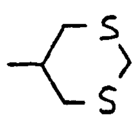 , 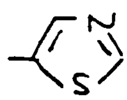 , 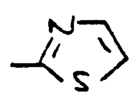 , 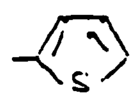 ,

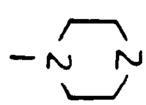

$-A^3$

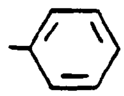 , 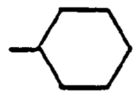 , 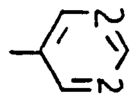 , 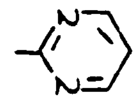 ,

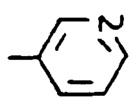 , 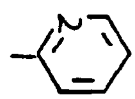 , 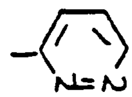

$-M^1$, $-M^2$ -CO-O, -O-CO, $-CH_2CH_2$, -CH=CH, $-CH_2O$, $-OCH_2$

X O oder S.

In einer bevorzugten Ausführungsform haben die Symbole in der allgemeinen Formel (I) die folgende Bedeutung:

$R^1$ ein geradkettiger oder verzweigter Alkyl- oder Alkenylrest mit 4 bis 14 C-Atomen, der ein asymmetrisches C-Atom enthalten kann, oder wobei eine $-CH_2$-Gruppe durch -O-, -CO-, -CO- oder -CO-O- ersetzt sein kann, oder wobei ein oder mehrere H durch F ersetzt sein können

j und l null oder 1

k, m, n null oder 1.

In einer weiteren bevorzugten Ausführungsform werden Tetrahydrofuran-2-carbonsäureester der allgemeinen Formel (IV) eingesetzt

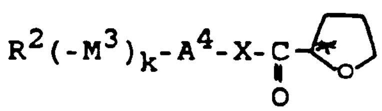 (IV)

worin bedeuten:

$R^2$ einen geradkettigen oder verzweigten Alkyl- oder Alkenylrest mit 6 bis 12 C-Atomen, der ein asymmetrisches C-Atom enthalten kann;

$-M^3$ -O, -S, -O-CO oder -CO

$-A^4$

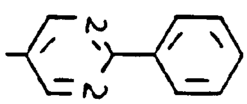 , 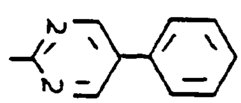 , 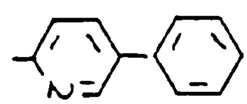 ,

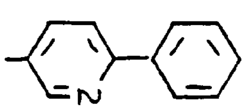 , 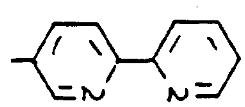 , 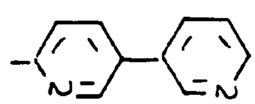 ,

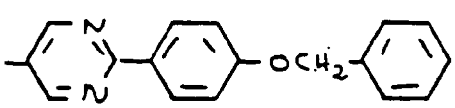 , 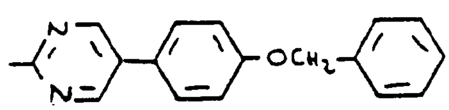 ,

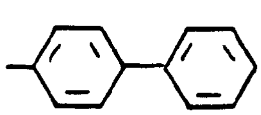 , 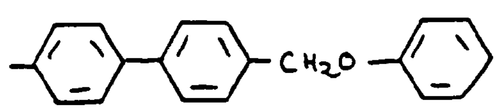 ,

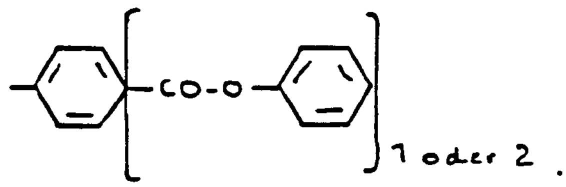

Zur Herstellung der Verbindungen der allgemeinen Formel (I) werden mesogene Phenole bzw. Thiophenole der allgemeinen Formel (II)

$$R^1(-A^1)_j(-M^1)_k(-A^2)_l(-M^2)_m(-A^3)_n-X-H \quad (II)$$

mit Derivaten der Tetrahydrofuran-2-carbonsäure der Formel (III)

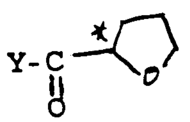

(III)

umgesetzt, wobei Y eine OH-Gruppe oder Halogen bedeutet. Für Y = OH erfolgt die Veresterung mit (II) in Gegenwart von Brönstedt- oder Lewis-Säuren, ggf. in Gegenwart wasserbindender Mittel, oder mit Kondensationsreagenzien wie N,N'-Carbonyldiimidazol, Dicyclohexylcarbodiimid oder Azodicarbonsäureester/Triphenylphosphin. Für Y = Halogen erfolgt die Umsetzung mit (II) in Gegenwart von Säurefängern insbesondere Pyridin oder Triethylamin. Schließlich können auch die Alkali- oder Erdalkalisalze von (II) mit den Säurehalogeniden [(III) mit Y = Halogen] zu (I) umgesetzt werden. Das Reaktionsprodukt kann durch an sich bekannte Maßnahmen, z.B. Umkristallisieren oder chromatographische Trennverfahren, gereinigt werden.

Phenol- bzw. Thiophenolkörper der Formel (II) sind bekannt. Ebenso sind die Methoden zur Herstellung von optisch aktiver Tetrahydrofuran-2-carbonsäure bekannt [z.B. P.C. Belander et al., Can. J. Chem. 61, 1383 (1983); H.B. Kagan et al., in "Topics in Stereochemistry" (E.L.Eliel, S.H. Wilen, Eds.) Vol 18, p. 249 f., Wiley Interscience, New York, Chichester, Brisbane, Toronto, Singapore, 1988].

Die Flüssigkristallmischungen gemäß der Erfindung bilden Flüssigkristall-Phasen und enthalten mindestens einen optisch aktiven Tetrahydrofuran-2-carbonsäureester.

Unter dem Begriff "Flüssigkristall-Phase" sind nematische, cholesterische, orthogonal smektische oder geneigt ("tilted")-smektische, insbesondere $S_A^*$-, $S_B^*$- und $S_C^*$-Phasen, zu verstehen. Die Flüssigkristallmischungen bestehen aus 2 bis 20, vorzugsweise 2 bis 15 Komponenten, darunter mindestens einer der erfindungsgemäß beanspruchten chiralen Verbindungen.

Die anderen Bestandteile werden vorzugsweise ausgewählt aus den bekannten Verbindungen mit nematischen, cholesterischen und/oder smektischen, z.B. $S_A$-Phasen, und/oder geneigt smektischen Phasen; dazu gehören beispielsweise Schiffsche Basen, Biphenyle, Terphenyle, Phenylcyclohexane, Cyclohexylbiphenyle, N-, S- oder O-haltige Heterocyclen (z.B. Pyrimidine), Zimtsäureester, Cholesterinester, verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Von der oder den erfindungsgemäßen Verbindung(en) enthalten die Flüssigkristallmischungen im allgemeinen 0,05 bis 70 Gew.-%, insbesondere 0,1 bis 50 Gew.-%.

Die erfindungsgemäßen Verbindungen sind insbesondere als Dotierstoffe für geneigt-smektische Flüssigkristallphasen geeignet, da sie diese in ferroelektrische Flüssigkristall-Phasen umwandeln; die Werte für die spontane Polarisation ($P_s$) liegen bei 10 Mol-% Dotierung und bei 25°C im Bereich von etwa 15-35 nC/cm² und im Bereich von etwa 150-350 nC/cm² linear extrapoliert auf die reine Verbindung; teilweise liegen die Werte für $P_s$ sogar noch höher.

Die Schaltzeiten der neuen Systeme liegen häufig unter 100 µs bei 10 Mol-% Dotierung, 25°C und einer Schaltspannung von ± 10 V/µm. Die erfindungsgemäßen Verbindungen können auch zur Erzielung des elektroklinen Effektes in orthogonalen smektischen Phasen , $S_A^*$, $S_B^*$, $S_E^*$) eingesetzt werden.

**Beispiel 1**

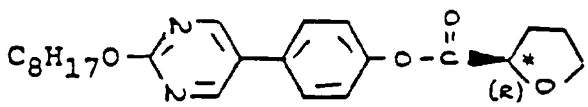

(R)-Tetrahydrofuran-2-carbonsäure-[4-(2-n-octyloxypyrimidin-5-yl)phenyl]-ester

Eine Lösung von 825 mg (4 mmol) Dicyclohexylcarbodiimid in 20 ml trockenem Dichlormethan wird unter Rühren bei Raumtemperatur mit 901 mg (3 mmol) 4-(2-n-Octyloxypyrimidin-5-yl)phenol, 464,5 mg (4 mmol) (R)-Tetrahydrofuran-2-carbonsäure sowie 49 mg (0,4 mmol) 4-Dimethylaminopyridin versetzt. Nach 10-stündigem Rühren bei Raumtemperatur wird die Reaktionslösung im Vakuum eingeengt. Chromatographische Reinigung und Umkristallisation aus n-Hexan ergeben 710 mg (59 %) farblose Kristalle mit $[\alpha]_D^{25}$ = -8,8° (c = 2, $CHCl_3$)

Schmelzpunkt: 75°C

Analog werden erhalten

**Beispiel 2**

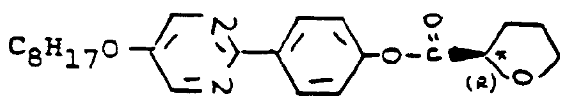

(R)-Tetrahydrofuran-2-carbonsäure-[4-(5-n-octyloxypyrimidin-2-yl)-phenyl]ester

$[\alpha]_D$ = 9,2° (c = 2, $CHCl_3$)

Schmelzpunkt: 90°C

**Beispiel 3**

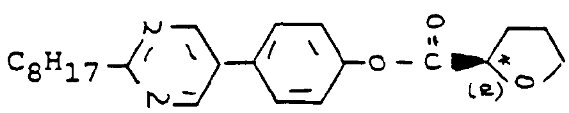

(R)-Tetrahydrofuran-2-carbonsäure-[4-(2-n-octylpyrimidin-5-yl)-phenyl]-ester

$[\alpha]_D$ = -9,3° (c = 2, $CHCl_3$)

Schmelzpunkt: 68°C

**Beispiel 4**

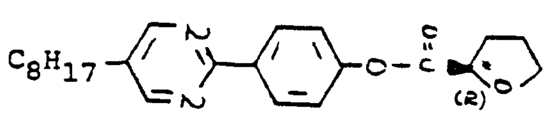

(R)-Tetrahydrofuran-2-carbonsäure-[4-(5-n-octylpyrimidin-2-yl)-phenyl]-ester

$[\alpha]_D$ = -9,8° (c = 2, $CHCl_3$)

Schmelzpunkt: 74°C

**Beispiel 5**

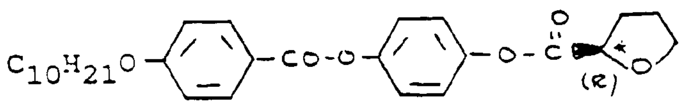

(R)-Tetrahydrofuran-2-carbonsäure-[4-(4-n-decyloxybenzoyl)-phenyl]-ester
$[\alpha]_D$ = -6,1° (c = 2, $CHCl_3$)
Schmelzpunkt: 80°C

**Beispiel 6**

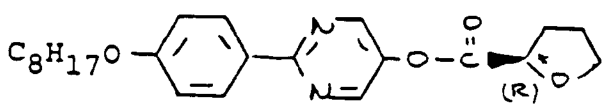

(R)-Tetrahydrofuran-2-carbonsäure-[2-(4-n-octyloxyphenyl)-pyrimidin-5-yl]-ester
$[\alpha]_D$ = -8.2° (c = 2, $CHCl_3$)
Schmelzpunkt: 90°C

**Beispiel 7**

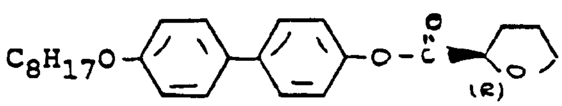

(R)-Tetrahydrofuran-2-carbonsäure-(4-n-octyloxybiphenyl-4-yl)-ester
$[\alpha]_D$ = -9,4° (c = 2, $CHCl_3$)
Schmelzpunkt: 119°C

**Beispiel 8**

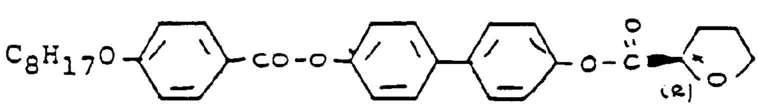

(R)-Tetrahydrofuran-2-carbonsäure-[4-(4-n-octyloxybenzoyloxy)-biphenyl-4′-yl]-ester
$[\alpha]_D$ = -6,9° (c = 2, $CHCl_3$)
Phasenfolge: K 121 (105 $S_X^*$ 106 $S_C^*$ 116) N∗ 195 I
Schmelzpunkt: 73°C

**Beispiel 9**

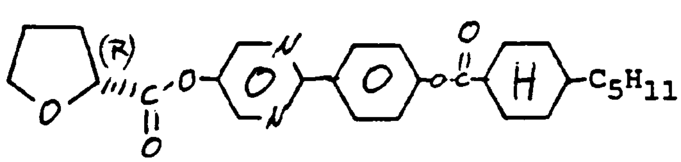

(R)-Tetrahydrofuran-2-carbonsäure-[2-(4-trans-pentyl) cyclohexyl-carbonyloxyphenyl)-pyrimidin-5-yl)-ester

$[\alpha]_D^{25}$ = -3,08° (c = 2, $CHCl_3$)

Phasenfolge: Fp 153 X 206-207°C I

**Beispiel 10**

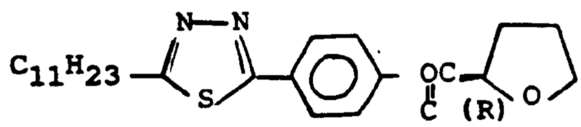

(R)-Tetrahydrofuran-2-carbonsäure-[4-(5-n-undecyl-1,3,4-thiadiazol-2-yl)-phenyl]-ester

$[\alpha]_D^{25}$ = -9,88° (c = 2, $CHCl_3$)

Phasenfolge: Fp 91-93°C

**Beispiel 11**

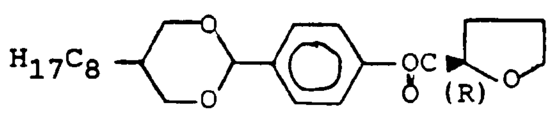

(R)-Tetrahydrofuran-2-carbonsäure-[4-(5-n-octyl-1,3-dioxan-2-yl)-phenyl]-ester

$[\alpha]_D^{25}$ = -5,16° (c = $CHCl_3$)

Phasenfolge: Fp 94-95°C

**Anwendungsbeispiele A1 bis A7**

Zur Überprüfung der Wirksamkeit der vorstehend beschriebenen Verbindungen als ferroelektrische Dotierstoffe in Flüssigkristallsystemen mit geneigten smektischen Phasen werden diese in Konzentrationen von jeweils 10 Mol-% mit einer nicht-chiralen Grundmischung (A) der Phasenfolge

K 12,5 $S_C$ 83 $S_A$ 95 N 100 I (A)

gemischt.

Es wurden jeweils die Werte für die spontane Polarisation ($P_s$ in $nC \cdot cm^{-2}$), für die Schaltzeit $\tau$ (in $\mu s$) und für den optischen Neigungswinkel der $S_C$-Phase $\theta$ (in °) der Mischung bestimmt. Die $P_s$-Werte werden nach der Methode von H.

Diamant et al. (Rev. Sci. Instr., 28, 30, 1957) gemessen. Bei einer Zellenschichtdicke von ca. 2 $\mu m$ wird durch Orientierungsschichten einheitliche planare Orientierung der Flüssigkristalle in der $S_C^*$-Phase erreicht [SSFLC-Technik, Clark et al., Appl. Phys. Lett. 36, 899 (1980)]. Zur Bestimmung von $\tau$ und $\theta$ wird die Meßzelle auf dem Drehtisch eines Polarisationsmikroskops zwischen gekreuztem Analysator und Polarisator befestigt. Der Schaltwinkel $2\theta$ wird bei an der Meßzelle angelegtem statischen elektrischen Feld gemessen. Für positive und negative Polarität dieses Feldes wird die Meßzelle jeweils so lang gedreht bis minimaler Lichtdurchgang auftritt. Der Winkelunterschied zwischen den beiden so ermittelten Orientierungen ergibt den Schaltwinkel. Mit Hilfe einer Photodiode erfolgt die Bestimmung der Schaltzeit $\tau$ indem die Anstiegszeit des Lichtsignals von 10 auf 90 % Signalhöhe gemessen wird. Die Schaltspannung beträgt ± 10 V/$\mu m$. Neben den Werten für $P_s$, $\tau$, $2\theta$ ist der $S_C^*$-Bereich der jeweiligen Mischung angegeben; die Werte in Klammern geben dabei die unterkühlbare untere Temperaturgrenze des $S_C$-Bereichs an.

Die Bestimmung des Pitches Z und der Verdrillungskraft HTP in der cholesterischen Phase erfolgte, wie bei P. Kassubek et al., Mol. Cryst. Liq. Cryst., Vol. 8, Seite 305 bis 314, 1969 beschrieben, in einer Keilzelle mit Orientierungsschicht durch Ausmessen der Versetzungslinien unter dem Polarisationsmikroskop.

Meßmethode: Versetzt man ein (nicht-chirales) Lösemittel mit einer kleinen Menge einer chiralen Verbindung, so wird die Ebene des linear polarisierten Lichts um den (charakteristischen) Winkel $\alpha$ gedreht; dieser Winkel wird wie folgt angegeben: $[\alpha]_D^T$ (c = x, LM), wobei die Symbole folgende Bedeutung haben: x = Konzentration der Lösung in g/l, LM = Lösemittel, D = 589 nm (NaD-Linie), T = Temperatur der Lösung. Der Drehwinkel wird in einem Polarimeter nach 10 cm Durchgang des Lichts bestimmt.

Tabelle 1 faßt die Ergebnisse zusammen.

Tabelle 1

| Anwendungs-Beispiel | (Substanz-) Beispiel | $S_C^*$-Bereich in Mischung (A)/°C | | $P_s$ (1) nC/cm² | τ (1) µs | 2θ (1) Grad | HTP (2) µm⁻¹ |
|---|---|---|---|---|---|---|---|
| A 1 | 1 | [-8] | 12-77 | 16 | 100 | 27 | 3 |
| A 2 | 2 | [-6] | 14-77,5 | 17 | 15 | 25 | 1,5 |
| A 3 | 3 | [-9] | 27-71 | 16 | 115 | 26 | 3,5 |
| A 4 | 4 | [-11] | 10-66 | 35 | 60 | 26 | 1,9 (4) |
| A 5 | 6 | [-10] | 14-76 | 15 | 120 | 31 | 1,6 |
| A 6 | 7 | [-2] | 15-80 | 10 (3) | 40 (3) | 24 (3) | 1,8 |
| A 7 | 8 | [0] | 15-83 | 4 (3) | 95 (3) | 22 (3) | 0,1 |

(1) Meßwerte bei 25°C

(2) Meßwerte bei 95°C

(3) Meßwerte bei 50°C

(4) Meßwerte bei 85°C

**Anwendungsbeispiel A8**

Eine ferroelektrische Mischung aus den Komponenten

| | | |
|---|---|---|
| flüssigkristalline Grundmischung A | 90 | Mol-% |
| (R)-Tetrahydrofuran-2-carbonsäure-[4-(2-n-Octyloxypyrimidin-5-yl)-phenyl]-ester | 5,93 | Mol-% |
| (2S,3R)-2-[4-(5-Octyl-pyrimidin-2-yl)-phenyloxy]-methyl-3-butyl-oxiran | 3,38 | Mol-% |

zeigt folgende flüssigkristalline Phasenbereiche:

X 5 $S_C^*$ 72,5 $S_A^*$ 87 N* 98 I

Bei 25°C weist diese Mischung eine spontane Polarisation von 23 nC cm⁻² auf, und bei einem Schaltfeld von 10 V µm⁻¹ eine Schaltzeit von 42 µs auf. Der Schmelzpunkt dieser Mischung liegt um 7°C unterhalb des Schmelzpunktes der Grundmischung A. Der Pitch dieser Mischung ist bei 90°C größer als 21 µm.

**Anwendungsbeispiel A9**

Eine ferroelektrische Mischung besteht aus den Komponenten

| | | |
|---|---|---|
| Grundmischung A | 90 | Mol-% |
| (R)-[2-(4'-Dodecyloxyphenyl)-pyrimidin-5-yl]-2,2-dimethyl-1,3-dioxolan-4-carbonsäureester | 6,07 | Mol-% |
| (R)-Tetrahydrofuran-2-carbonsäure-[4-(2-n-octyloxypyrimidin-5-yl)-phenyl]-ester | 3,93 | Mol-% |

und weist folgende flussigkristalline Phasenbereiche auf

X 7,4 $S_C^*$ 81 $S_A^*$ 86 N* 100 I

Bei 25°C weist diese Mischung eine spontane Polarisation von 18 nC $cm^{-2}$, und bei einem Schaltfeld von 10 V $\mu m^{-1}$ eine Schaltzeit von 51 µs auf. Der Schmelzpunkt dieser Mischung liegt um 5,5°C unter dem der Grundmischung A. Der Pitch dieser Mischung ist bei einer Temperatur von 90°C größer als 40 µm.

Anhand der Anwendungsbeispiele A8 und A9 erkennt man, daß die Tetrahydrofuran-Derivate auch in Kombination mit anderen Dotierstoffen zu sehr guten ferroelektrischen Mischungen führen, die aufgrund des großen Pitches gut orientierbar sind und kurze Schaltzeiten aufweisen.

**Anwendungsbeispiel A10**

Einer ferroelektrischen flüssigkristallinen Mischung bestehend aus

| | | |
|---|---|---|
| 5-Octyloxy-2-[4-hexyloxyphenyl]pyrimidin | 12,5 | Mol-% |
| 5-Octyloxy-2-[4-octyloxyphenyl]pyrimidin | 4,1 | " |
| 5-Octyloxy-2-[4-butyloxyphenyl]pyrimidin | 13,9 | " |
| 5-Octyloxy-2-[4-decyloxyphenyl]pyrimidin | 7,6 | " |
| 5-Octyloxy-2-[4-dodecyloxyphenyl]pyrimidin | 7,6 | " |
| 5-Octyloxy-2-[4-dodecylphenyl]pyrimidin | 11,2 | " |
| trans-4-pentyl-cyclohexancarbonsäure-[4-(5-decyl-pyrimidin-2-yl)]-phenylester | 17,75 | " |
| 4-[5-Octylpyrimidin-2-yl]phenylhexancarbonsäureester | 4,1 | " |
| 4-(Nonyloxypyrimidin-5-yl)-phenyl-2-chlor-4-methyl-pentansäureester (Racemat) | 4,1 | " |
| (R)-4-(5-n-Octyloxypyrimidin-2-yl)-phenyl-(2,2 pentamethylen-1,3-dioxolan-4-yl)methylether | 4,5 | " |
| (2R,3R)-3-Propyl-oxiran-2-carbonsäure-[4-(2-octyloxy-pyrimidin-5-yl)phenyl]-ester | 4,35 | " |
| und | | |
| (R)-Tetrahydrofuran-2-carbonsäure-[4-(5-n-octylpyrimidin-2-yl)-phenyl]ester | 8,3 | " |

weist folgende Phasenbereiche auf

X -8 $S_C^*$ 66 $S_A^*$ 73 N* 81 I

Die Mischung weist bei 25°C eine Polarisation von 38 nC $cm^{-2}$ auf; die Temperaturabhängigkeit des Pitches ist in der folgenden Tabelle wiedergegeben:

| T/°C | 73 | 74 | 75 | 76 | 77 | 78 | 79 | 80 | 81 |
|---|---|---|---|---|---|---|---|---|---|
| Z/µm | +22 | +29 | +53 | >100 | ∞ | >100 | -42 | -32 | -25 |

Offensichtlich sind die Tetrahydrofuran-Derivate u.a. für ferroelektrische Mischungen einsetzbar, die trotz der sehr hohen Polarisation im gesamten nematischen Phasenbereich einen sehr großen Pitch aufweisen und sich daher gut orientieren lassen.

## Patentansprüche

1. Verwendung von optisch aktiven Tetrahydrofuran-2-carbonsäureestern als Dotierstoffe in Flüssigkristallmischungen.

2. Verwendung nach Anspruch 1, dadurch gekennzeichnet, daß Tetrahydrofuran-2-carbonsäureester der allgemeinen Formel I verwendet werden

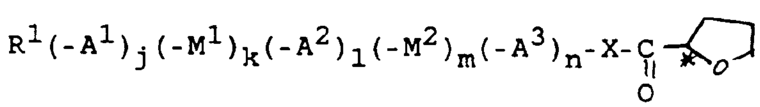 (I)

in der die Symbole und Indices folgende Bedeutung haben:
$R^1$

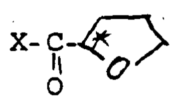

oder ein geradkettiger oder verzweigter
Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, eine oder mehrere nicht benachbarte $-CH_2$-Gruppen durch -O-, -S-, -CO-, -O-CO- und/oder -CO-O- ersetzt sein können, und wobei ein oder mehrere H durch F, Cl, Br oder CN ersetzt sein können
j und l null, 1 oder 2
k und m null oder 1
n null, 1 oder 2
mit folgender Maßgabe: wenn j und/oder l = null sind, ist k = null; wenn n = null ist, ist m = null; die Summe j + l + n ist minimal 1 und maximal 3,
$-A^1$, $-A^2$

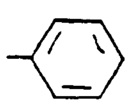 , 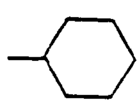 , 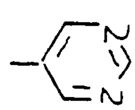 , 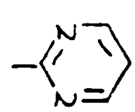 ,

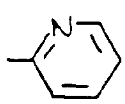 , 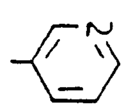 , 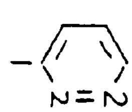 , 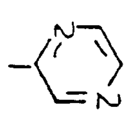 ,

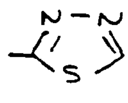 , 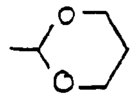 , 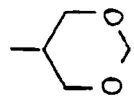 , 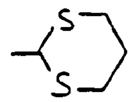 ,

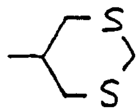 , 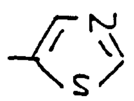 , 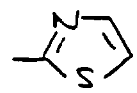 , 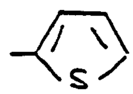 ,

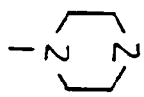

-$A^3$

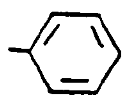 , 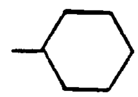 , 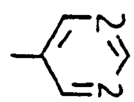 , 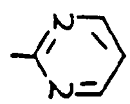 ,

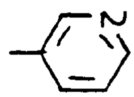 , 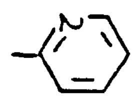 , 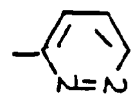 .

-$M^1$, -$M^2$ -CO-O, -O-CO, -$CH_2CH_2$, -CH=CH, -$CH_2O$, -$OCH_2$
X O oder S.

3. Verwendung nach Anspruch 2, dadurch gekennzeichnet, daß Tetrahydrofuran-2-carbonsäureester der allgemeinen Formel (IV) verwendet werden

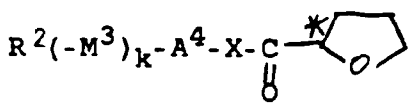 (IV)

worin bedeuten:

$R^2$ einen geradkettigen oder verzweigten Alkyl- oder Alkenylrest mit 6 bis 12 C-Atomen, der ein asymmetrisches C-Atom enthalten kann;
-$M^3$ -O, -S, -O-CO oder -CO
-A4

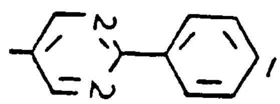 , 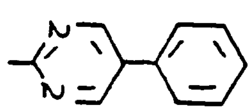 , 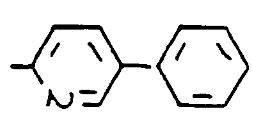 ,

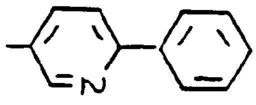 , 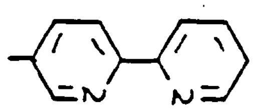 , 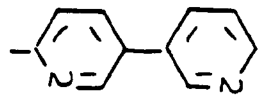 ,

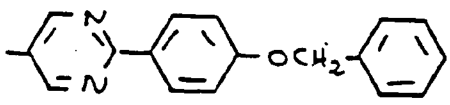 , 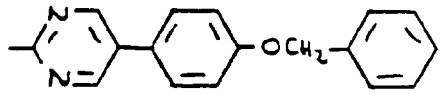 ,

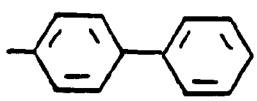 , 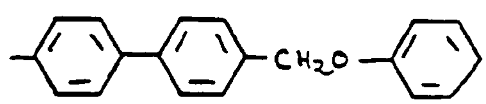 ,

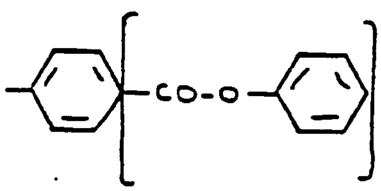 1 oder 2.

4. Optisch aktive Tetrahydrofuran-2-carbonsäureester der allgemeinen Formel (I) nach Anspruch 2.

5. Optisch aktive Tetrahydrofuran-2-carbonsäureester der allgemeinen Formel (IV) nach Anspruch 3.

6. Flüssigkristallmischung, gekennzeichnet durch einen Gehalt an mindestens einem optisch aktiven Tetrahydrofuran-2-carbonsäureester.

7. Flüssigkristallmischung, gekennzeichnet durch einen Gehalt an mindestens einem optisch aktiven Tetrahydrofuran-2-carbonsäureester der allgemeinen Formel (I) nach Anspruch 2.

8. Flüssigkristallmischung, gekennzeichnet durch einen Gehalt an mindestens einem optisch aktiven Tetrahydrofuran-2-carbonsäureester der allgemeinen Formel (IV) nach Anspruch 3.

9. Elektrooptisches Schalt- oder Anzeigeelement enthaltend eine Flüssigkristallmischung nach Anspruch 6.

10. Verfahren zur Herstellung von optisch aktiven Tetrahydrofuran-2-carbonsäureestern der allgemeinen Formel (I) nach Anspruch 2, dadurch gekennzeichnet, daß man mesogene Phenole bzw. Thiophenole der allgemeinen Formel (II) mit Derivaten der Tetrahydrofuran-2-carbonsäure der allgemeinen Formel (III) umsetzt, wobei Y eine OH-Gruppe oder Halogen bedeutet:

$$R^1(-A^1)_j(-M^1)_k(-A^2)_l(-M^2)_m(-A^3)_n-X-H, \quad (II)$$

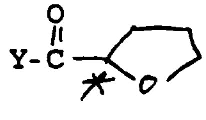 . (III)

## Claims

1. The use of optically active tetrahydrofuran-2-carboxylic acid esters as dopants in liquid-crystal mixtures.

2. The use as claimed in claim 1, wherein use is made of tetrahydrofuran-2-carboxylic acid esters of the gen-

eral formula (I)

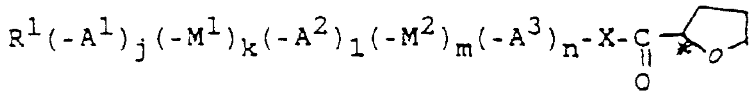 (I)

in which the symbols and indices have the following meaning:
$R^1$ is

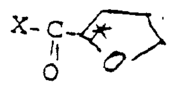

or a straight-chain or branched alkyl radical containing 1 to 16 carbon atoms or a straight-chain or branched alkenyl radical containing 3 to 16 carbon atoms, it being possible for these radicals themselves to contain asymmetric carbon atoms, for one or more non-adjacent $-CH_2$-groups to be replaced by -O-, -S-, -CO-, -O-CO-and/or -CO-O-, and for one or more hydrogen atoms to be replaced by fluorine, chlorine, bromine or CN,
j and l are zero, 1 or 2,
k and m are zero or 1,
n is zero, 1 or 2,
with the following proviso: if j and/or 1 = zero, k = zero; if n = zero, m = zero; the sum of j + l + n is not less than 1 and not more than 3,
$-A^1$, $-A^2$ is

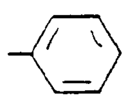 , 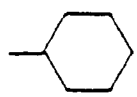 , 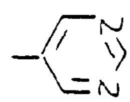 , 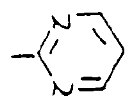 ,

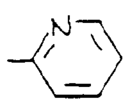 , 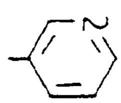 , 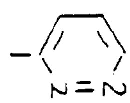 , 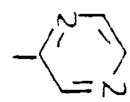 ,

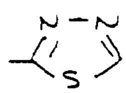 , 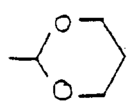 , 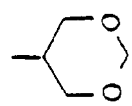 , 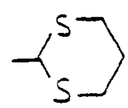 ,

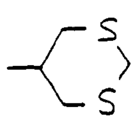 , 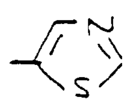 , 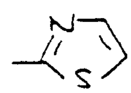 , 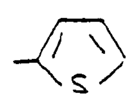 ,

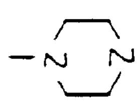

$-A^3$ is

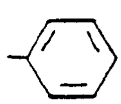 , 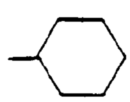 , 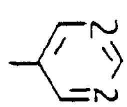 , 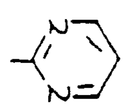 ,

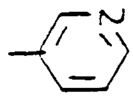 , 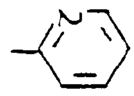 , 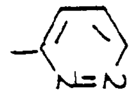

$-M^1$, $-M^2$ is -CO-O, -O-CO, $-CH_2CH_2$, -CH=CH, $-CH_2O$, - $OCH_2$ and X is O or S.

**3.** The use as claimed in claim 2, wherein use is made of tetrahydrofuran-2-carboxylic acid esters of the general formula (IV):

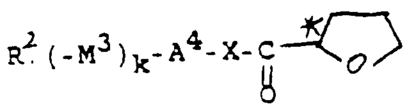

in which:

$R^2$ denotes a straight-chain or branched alkyl or alkenyl radical containing 6 to 12 carbon atoms, which may contain an asymmetric carbon atom;

$-M^3$ denotes -O, -S, -O-CO or -CO

$-A^4$ denotes

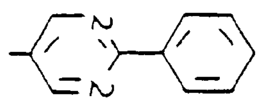 , 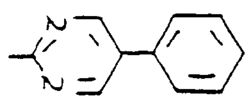 , 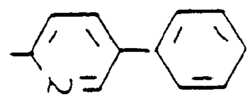 ,

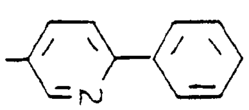 , 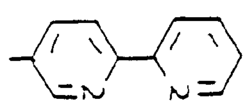 , 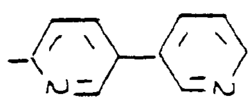 ,

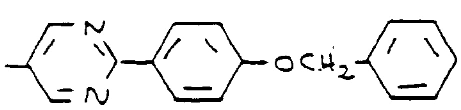 , 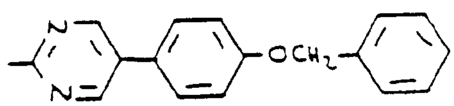 ,

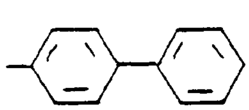 , 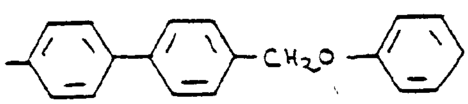 ,

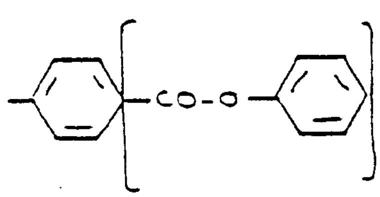 1 or 2.

**4.** An optically active tetrahydrofuran-2-carboxylic acid ester of the general formula (I) as claimed in claim 2.

**5.** An optically active tetrahydrofuran-2-carboxylic acid ester of the general formula (IV) as claimed in claim 3.

**6.** A liquid-crystal mixture which contains at least one optically active tetrahydrofuran-2-carboxylic acid ester.

7. Liquid-crystal mixture which contains at least one optically active tetrahydrofuran-2-carboxylic acid ester of the general formula (I) as claimed in claim 2.

8. Liquid-crystal mixture which contains at least one optically active tetrahydrofuran-2-carboxylic acid ester of the general formula (IV) as claimed in claim 3.

9. Electro-optic switching or display component containing a liquid-crystal mixture as claimed in claim 6.

10. Process for preparing an optically active tetrahydrofuran-2-carboxylic acid ester of the general formula (I) as claimed in claim 2, comprising reacting mesogenic phenols or thiophenols of the general formula (II) with derivatives of the tetrahydrofuran-2 -carboxylic acid of the general formula (III), Y denoting an OH group or halogen:

$$R^1(-A^1)_j(-M^1)_k(-A^2)_l(-M^2)_m(-A^3)_n-X-H, \quad (II)$$

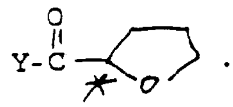 (III)

## Revendications

1. Utilisation d'esters optiquement actifs de l'acide tétrahydrofuranne-2-carboxylique comme matières de dopage dans des mélanges à propriétés de cristaux liquides.

2. Utilisation selon la revendication 1, caractérisée en ce que l'on utilise des esters de l'acide acide tétrahydrofuranne-2-carboxylique répondant à la formule générale I

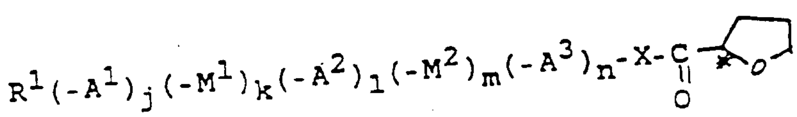 (I)

dans laquelle les symboles et indices ont le sens suivant :
$R^1$ représente

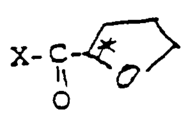

ou un reste alkyle linéaire ou ramifié ayant 1 à 16 atomes de carbone ou un reste alcényle, linéaire ou ramifié, ayant 3 à 16 atomes de carbone, ces restes pouvant contenir eux-mêmes des atomes de C asymétrique, un ou plusieurs des groupes $-CH_2-$, qui ne sont pas voisins, peuvent être remplacés par -O-, -S-, -CO-, -O-CO- et/ou -CO-O-, et un ou plusieurs H peut ou peuvent être remplacés par F, Cl, Br ou CN,

j et l valent chacun 0,1 ou 2

k et m valent chacun 0 ou 1

N vaut 0,1 ou 2

à la condition que, lorsque j ou/l est ou sont nuls, k est nul; lorsque n vaut 0, m est nul; la somme (j+l+n) vaut au minimum 1 et au maximum 3;

$A^1$, et $A^2$ représentent chacun :

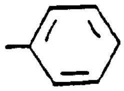 , 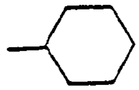 , 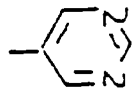 , 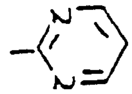 ,

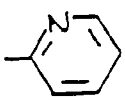 , 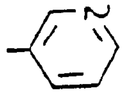 , 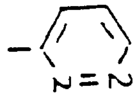 , 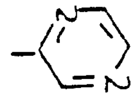 ,

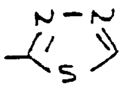 , 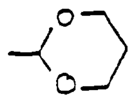 , 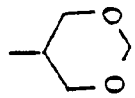 , 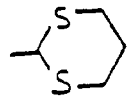 ,

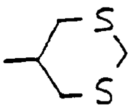 , 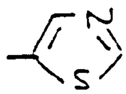 , 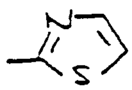 , 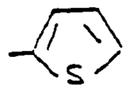 ,

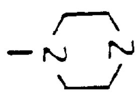

-$A^3$ représente

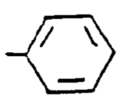 , 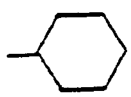 , 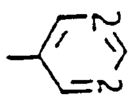 , 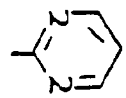 ,

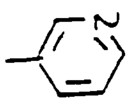 , 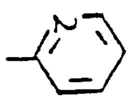 , 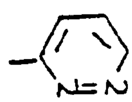 .

$M^1$ et $M^2$ représentent chacun -CO-O, -O-CO, - $CH_2CH_2$, -CH=CH, -$CH_2O$, -$OCH_2$;
X représente O ou S.

3. Utilisation selon la revendication 2, caractérisée en ce que l'on utilise des esters d'acide tétrahydrofurane-2-carboxylique répondant à la formule générale (IV) :

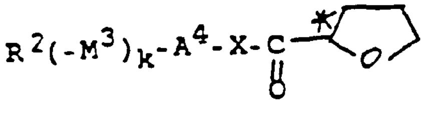 (IV)

dans laquelle :

$R^2$ représente un reste alkyle ou alcényle, linéaire ou ramifié, ayant 6 à 12 atomes de carbone, qui peut contenir un atome de C asymétrique;

-$M^3$ représente -O, -S, -O-CO ou -CO

-$A^4$ représente

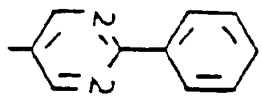,
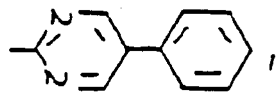,
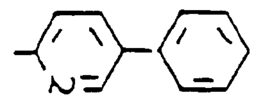,

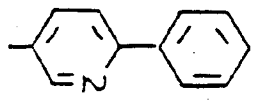,
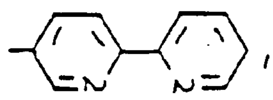,
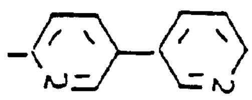,

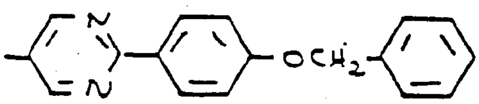,
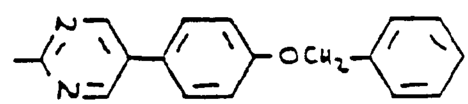,

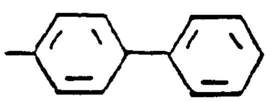,
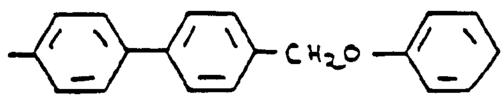,

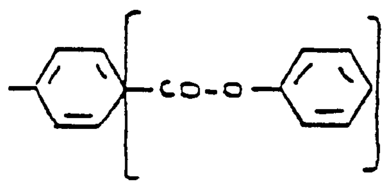
1 ou 2.

4. Esters optiquement actifs de l'acide tétrahydrofuranne-2-carboxylique, de formule générale (I) selon la revendication 2.

5. Esters optiquement actifs de l'acide tétrahydrofuranne-2-carboxylique de formule générale (IV) selon la revendication 3.

6. Mélange à propriétés de cristaux liquides, caractérisé par une teneur en au moins un ester optiquement actif de l'acide tétrahydrofuranne-2-carboxylique.

7. Mélange à propriétés de cristaux liquides, caractérisé par une teneur en au moins un ester optiquement actif de l'acide tétrahydrofuranne-2-carboxylique répondant à la formule générale (I) selon la revendication 2.

8. Mélange à propriétés de cristaux liquides, caractérisé par une teneur en au moins un ester optiquement actif de l'acide tétrahydrofuranne-2-carboxylique de formule générale (IV) selon la revendication 3

9. Elément électro-optique de commutation ou d'affichage, contenant un mélange à propriétés de cristaux liquides selon la revendication 6.

10. Procédé pour préparer des esters optiquement actif de l'acide tétrahydrofuranne-2-carboxylique, de formule générale (I) selon la revendication 2, caractérisé en ce qu'on fait réagir des phénols ou thiophénols mésogènes de formule générale (II) avec des dérivés de l'acide tétrahydrofuranne-2-carboxylique de formule (III), dans laquelle Y représente un groupe OH ou un atome d'halogène :

$$R^1(-A^1)_j(-M^1)_k(-A^2)_l(-M^2)_m(-A^3)_n-X-H, \quad (II)$$

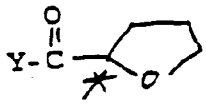

(III)